# EUROPÄISCHE PATENTANMELDUNG

(11) **EP 1 987 841 A1**
(43) Veröffentlichungstag der Anmeldung: **05.11.2008**
(21) Anmeldenummer: 08009600.1
(22) Anmeldetag: 03.04.2003
(51) Int. Cl.: A61K 39/39, A61P 31/00

(54) **Verwendung eines Lipopeptids oder Lipoproteins als Adjuvans bei therapeutischer oder prophylaktischer Vakzinierung**

(30) Priorität: 04.04.2002 EP 02007640
(62) Teilanmeldung aus: 03745782.7
(71) Anmelder: Helmholtz-Zentrum für Infektionsforschung GmbH, 38124 Braunschweig (DE)
(72) Erfinder: Guzman, Carlos Alberto, 38304 Wolfenbüttel (DE); Mühlradt, Peter, 38114 Braunschweig (DE)
(74) Vertreter: Kröncke, Rolf

(57) **Zusammenfassung**

Es wird die Verwendung von Lipopeptiden und Lipoproteinen als mukosale Adjuvantien für verschiedenste Vakzinierung über die Schleimhäute vorgeschlagen, insbesondere auf intranasalem Wege. Bei den Lipopeptiden handelt es sich um an der aminoterminalen Cystein eines Peptids oder Proteins 2,3-diacyloxy(2R)-propyl-substituierte Peptide oder Proteine, bevorzugt aus Mykoplasmen abgeleitete S-(2,3-bispalmitoyloxy-(2R)-propyl)cysteinyl-peptide. Die angegebenen Peptide sind bereits in geringen Dosen hochwirksam und führen zu guten Immunisierungsergebnissen und Erhöhung u.a. des IgA-Spiegels.

## Beschreibung

Die Erfindung betrifft die Verwendung eines Lipopeptids oder Lipoproteins als Adjuvans bei therapeutischer oder prophylaktischer Vakzinierung, insbesondere über die Schleimhäute, d.h. als Mucosal-Adjuvans.

Ein Drittel aller jährlichen Todesfälle in der Welt werden noch immer durch Infektionskrankheiten verursacht, die außerdem für wenigstens 15 % der Krebsneuerkrankungen verantwortlich sind. Infektionskrankheiten sollen ferner in die Pathophysiologie verschiedener chronischer Krankheiten entzündlicher, vaskulärer oder degenerativer Art verwickelt sein. Infektionskrankheiten verursachen der Allgemeinheit hohe Kosten durch Behandlungskosten und Arbeitsausfall der Erkrankten.

Zur Abwehr von Infektionskrankheiten werden generell zwei Wege verfolgt, nämlich Therapie und Prophylaxe. Hierbei sind Impfungen zur wirksamsten Waffe gegen Infektionskrankheiten geworden. Es gibt jedoch noch viele Infektionskrankheiten, für die noch keine Vakzine zur Verfügung stehen oder eine hinreichende Immunisierung nicht erreicht werden kann. Viele Vakzine sind unzureichend wegen geringer Effizienz, schweren Nebenwirkungen, geringer Stabilität oder hoher Kosten. Es besteht daher ein starkes Bedürfnis für neue und verbesserte Impfstoffe (Vakzine).

Traditionell wurden Vakzine für die Prophylaxe bei Infektionskrankheiten angewendet und sind auf diesem Gebiet für viele Krankheiten gut eingeführt. Neuere Erkenntnisse legen nahe, dass Vakzinierungen außerdem ein sehr geeignetes Mittel bei der Immuntherapie anderer ansteckender Krankheiten sind, bei denen bisher noch nicht geimpft wurde, wie viraler Hepatitis, Helicobacter-pylori-Infektionen, Herpesvirus-Infektionen usw.. Ein weiteres Einsatzgebiet ist die Einbindung von Vakzinen bei Immuntherapien und Immunprophylaxen gegen Autolmmunkrankheiten, Entzündungskrankheiten, Tumoren, Allergien und zur Empfängnisverhütung bei Mensch und Tier. Teilweise (insbesondere auch im letztgenannten Fall) scheint die Verwendbarkeit von Vakzinen an einen effizienten mukosalen Verabreichungsweg und die damit verbundene Erzeugung einer guten mukosalen Immunreaktion gebunden zu sein.

Die meisten Infektionen sind entweder auf die Schleimhäute beschränkt oder die Krankheitsauslöser müssen während früher Infektionsphasen die Schleimhaut passieren. Es Ist daher unbedingt anzustreben, dass bei einer Vakzinierung nicht nur eine systemische sondern vor allem auch eine mukosale Immunantwort erhalten wird, um hierdurch sowohl die Infektion (Kolonisierung) als auch die Ausbildung der Krankheit primär zu stoppen. Eine gute mukosale Immunantwort würde die Ansteckungsgefahr deutlich herabsetzen.

Dadurch dass sich das systemische und das mucosale Immunsystem zwar teilweise überlappen, aber nicht identisch sind, sind parenteral verabreichte Vakzine für den Schutz gegen mucosal Pathogene weniger wirksam (McGhee, Mestecky et al., "The mucosal immune system: from fundamental concepts to vaccine development", Vaccine 10, 75-88 (1992)). Tatsächlich stimulieren parenteral verabreichte Vakzine im wesentlichen systemische Immunantworten, während auf mukosalem Weg, d.h. über die Schleimhäute, verabreichte Vakzine die Immunantwort nachbilden, wie sie durch natürliche Infektionen hervorgerufen wird. Auf diese Weise führt die mukosale Immunisierung zu wirkungsvollen systemischen und mukosalen Immunantworten.

Weiterhin ist zu erwarten, dass die mukosale Verabreichung von Vakzinen weniger Nebenwirkungen mit sich bringt und bei den Patienten gut akzeptiert wird. Auf mukosalem Weg sind Vakzine leichter zu verabreichen und besser in Übereinstimmung mit Vakzinierungsprotokollen zu bringen. Ihre Bereitstellung ist mit geringeren Kosten verbunden.

Die Verabreichung von Antigenen auf mukosalem Weg ist bisher mit einigen beträchtlichen Schwierigkeiten verbunden. Ein Hauptproblem besteht darin, dass die so verabreichten Antigene häufig kaum immunogen sind. Dies beruht auf unterschiedlichen Mechanismen wie (i) beschleunigte Antigen-Eliminierung durch nicht-spezifische Clearance-Mechanismen des Wirts (beispielsweise Zilienaktivität, Peristaltik), (ii) Antigen-Abbau durch lokal wirkende Enzyme, (iii) Antigen-Veränderung und/oder strukturelle Modifizierung infolge extremer pH-Werte (saures Milieu im Magen, alkalisches im Intestinaltrakt), (iv) schwache Antigen-Durchlässigkeit der Schleimhäute sowie (v) nur sehr begrenzter Zugang zu Antigenpräsentierenden Zellen.

Zur Überwindung dieser Schwierigkeiten werden verschiedene Strategien angewendet, z.B., Einschluss oder Assoziierung der Antigene mit Partikeln (Mikropartikeln. Nanopartikeln, Bakterien oder Bakterienfragmenten) als Trägem, Verwendung virenartiger Konstrukte, Verwendung von Liposomen oder ISCOMS (immunstimulatorische Komplexe) oder Virosomen, Verwendung transgener Pflanzen, Antigen-Produktion durch abgeschwächte virale oder bakterielle Träger, entweder als übliche Vektoren oder als Träger für Nukleinsäure-Vakzine und/oder die Verabreichung dieser Hilfsmittel mit mukosalen Adjuvantien. Trotz intensiver Bemühungen auf diesem Gebiet sind bisher praktisch keine hinlänglich wirksamen und gleichzeitig gut verträglichen mukosalen Adjuvantien zur Praxisreife geführt worden.

Als "Adjuvantien" werden Substanzen bezeichnet, die bei einer Immunisierung dem eigentlichen Antigen (d.h. der Substanz, die die gewünschte Immunreaktion provoziert) beigefügt werden, um die humorale und/oder zellvermittelte Immunantwort zu verstärken ("Lexikon der Biochemie und Molekularbiologie", 1. Band, Spektrum Akademischer Verlag¹⁹⁹⁵) Die Verwendung vieler Adjuvantien beruht allein auf Erfahrung, wobei die Wirkung weder genau zu erklären noch vorauszusagen ist. Traditionell werden vor allem folgende Gruppen von Adjuvantien verwendet: Aluminiumhydroxid, Emulsionen von Mineralölen, Saponine, Detergentien, Silizlumverbindungen, Thioharnstoff, Endotoxine gramnegativer Bakterien, Exotoxine grampositiver Bakterien, abgetötete Bakterien oder deren Teile.

Die Verwendung optimaler Adjuvantien spielt bei der Vakzinierung eine entscheidende Rolle. Ohne Adjuvans verabreichte Antigene vermitteln nur selten eine ausreichende Immunantwort. Darüberhinaus kommt es nicht nur auf die Stärke der hervorgerufenen Immunantwort an, sondern auch auf auf ihre Qualität. Die Stimulierung eines falschen lmmunisierungsmusters kann zu immunopatholagischen Reaktionen und Verschlechterung der Infektionssymptome führen. In diesem Zusammenhang kann das Adjuvans helfen, die gewünschte Immunreaktion zu unterstützen.

Die für den Menschen zugelassenen Adjuvantien sind,begrenzt. Eines der wenigen von den Zulassungsbehörden für den Menschen akzeptierten Adjuvantien ist Aluminiumhydroxid. Aus der Tatsache, dass ein Adjuvans bei systemischer Applikation aktiv ist, also die Wirkung eines Antigens unterstützt, lässt sich nicht darauf schließen, dass dies auch für andere Applikationswege gilt. Ein typisches Beispiel ist das Aluminiumhydroxid, welches die Immunogenität einer Substanz bei intramuskulärer, subkutaner, intraperitonealer oder intradermaler Gabe unterstützen kann, das jedoch bei mukosaler Gabe vollständig unwirksam bleibt.

In den letzten Jahren ist intensiv nach neuen Adjuvantien, auch solchen für den mukosalen Verabreichungsweg, gesucht worden. Nur wenige Stoffe wurden gefunden, die in der Lage sind Mukosalantworten zu verstärken. Unter diesen wirken einige als Träger, an die die Antigene gebunden oder mit diesen fusioniert werden müssen. Weit weniger wurden universell einsetzbare "echte" Adjuvantien gefunden, die den Antigenen zugemischt werden.

Als echte Mukosai-Adjuvantien wurden das hitzeinstabile Toxin aus *Escherichia coli* und das Choleratoxin aus *Vibrio cholerae* entdeckt. Von beiden wurde eine Wirksamkeit als Mukosal-Adjuvans beschrieben (Holmgren et al, "Cholera toxin and cholera B subunit as oral-mucosal adjuvant and antigenvector system", Vaccine 1179-1184, 1993 und Douce et al, "Mutants of Escherichla coli heat-labile toxin lacking ADP-ribosyltransferase activity act as nontoxic, mucosal adjuvants", Proc. Natl. Acad. Sci. USA 92, 1644-1648). Die ihnen innewohnende Toxizität und die potentiellen Nebenwirkungen beeinträchtigen jedoch ihre Verwendbarkeit im Zusammenhang mit Vakzinierungen beim Menschen. Obwohl auf gentechnischem Wege nicht-toxische Derivate dieser Moleküle erzeugt wurden, werden noch immer starke, nicht tolerierbare Nebenwirkungen berichtet, wie krankhafte Veränderungen der Respirationsschleimhaut und Eindringen des Toxins in das Gehim (N. Garçon, Präsentation auf dem "Word Vaccine Congress", Genf, 26. bis 28. September 1999; und: van Ginkel, F.B. et al., "cutting edge: The Mucosal Adjuvant Cholera Toxin Redirects Vaccine Proteins into Olfactory Tissues", J. Immunol. 2000, 165, 4778-4782).

Es besteht daher noch immer ein dringendes Bedürfnis für neue verträgliche und effektive Mukosal-Adjuvantien.

Der Erfindung lag daher die Aufgabe zugrunde, eine Palette neuer, hochwirksamer und für den Menschen nicht-toxischer Mukosal-Adjuvantien zu entwickeln, die mit den verschiedensten zu unterstützenden aktiven Bestandteilen in herkömmlichen oder neuartigen Vakzinen, wie insbesondere prophylaktischen oder therapeutischen Impfstoffen einschließlich Krebs- und DNA-Vakzinen, einsetzbar sind.

Zur Lösung dieser Aufgabe wird die Verwendung eines Lipopeptids oder Lipoproteins der Struktur (I) als Mukosal-Adjuvans bei therapeutischer oder prophylaktischer Vakzinierung über die Schleimhäute vorgesehen, 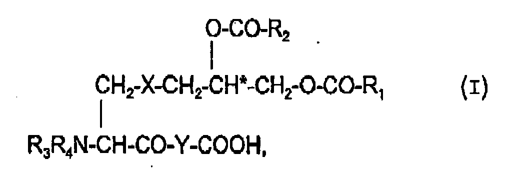 wobei
R₁ und R₂, die gleich oder voneinander verschieden sein können, für C₇₋₂₅-Alkyl, C₇₋₂₅-Alkenyl oder C₇₋₂₅-Alkinyl,
X für S, O oder CH₂,
R₃ und R₄ unabhängig voneinander für H oder Methyl und
Y für eine aus 1 bis 25 Aminosäureresten bestehende physiologisch verträgliche und in der verwendeten Spezies nicht per se immunogene Aminosäuresequenz stehen,
und das mit * markierte asymmetrische Kohlenstoffatom die absolute R-Konfiguration hat, wenn X = S (Schwefel) ist, gemäß der Regel von Cahn-Ingold-Prelog.

Zwar wurde in der DE 19652586 A1 bereits die Verwendung eines bestimmten S-(2,3-Dihydroxypropyl)-cystein-peptids mit zwei esterartig an die Dihydroxypropylgruppe gebundenen Fettsäuren als Vakzin-Adjuvans miterwähnt, jedoch rein hypothetisch und ohne. Bezug auf Mukosaladjuvantien, so dass von einer üblichen Vakzinierungsroute auszugehen ist.

Vorzugsweise ist die carboxyterminal an die 2,3-diacyloxypropyl-substituierte Aminosäure gebundene Aminosäuresequenz (Y) ausgewählt aus folgenden Sequenzen:
a) GQTNT,
b) SKKKK,
c) GNNDESNISFKEK oder
d) GQTDNNSSQSAAPGSGTT'NT..

Derzeit besonders bevorzugt ist ein S-[2,3-Bispalmitoyloxy-(2*R*)-propyl]cysteinyl-peptid, wobei die Peptidkette wiederum eine aus 1 bis 25 Aminosäuren bestehende physiologisch verträgliche, in der verwendeten Spezies nicht per se immunogene Aminosäuresequenz sein kann.

Nach derzeitiger Erkenntnis könnte die carboxyterminale Peptidkette eine die Hydrophilizität oder Lipophilizität des Lipopeptids steuernde und gegebenenfalls modifizierende Funktion haben, so dass generell alle Peptid- oder Proteinketten, die - je nach Einsatzzweck der Vakzine - dieses Kriterium erfüllen, verwendet werden können. Die Peptidkette soll physiologisch verträglich und insbesondere nicht selbst in der verwendeten Spezies (d.h. der geimpften (vakzinierten) Spezies, ob Mensch oder Tier) immunogen sein.

Als entscheidend für die Wirksamkeit als Mukosal-Adjuvans wird derzeit die Struktur gemäß Formel (I) angesehen, wobei dem asymmetrischen Zentrum an der bezeichneten Stelle eine zentrale Bedeutung zuzukommen scheint.

Das erfindungsgemäße Mukosaladjuvans kann mit allen dem Fachmann bekannten Methoden an das für die Vakzinierung vorgesehene. Antigen oder aktive Molekül gebunden, gemeinsam mit diesem in physikalische (z.B. Micropartikel, Nanopartikel, Liposomen, ISCOMS, Polymere) oder biologische Partikel (Bakterien, Bakterienteile) oder Virosomen inkorporiert oder mit dem Antigen gemischt werden. Bei der Bindung an Träger können auch Transportmoleküle oder Transportproteine als Träger vorgesehen sein.

Vorzugsweise liegt das erfindungsgemäß verwendete Lipoprotein oder Lipopeptid gemäß der o.a. Formel (I) in einer Zubereitung mit der aktiven Vakzinierungskomponente (z.B. dem Antigen) vor, die für intranasale, intra-NALT (nasal associated lymphoid tissue), aerosolisiert, orale, intrarektale, conjunctivale, intravaginale, intraurethrale Applikation oder für die Applikation in die Milchgänge der weiblichen Brust geeignet und vorgesehen ist. Alternativ kann das erfindungsgemäße Mukosal-Adjuvans in einem Kit für die Co-Applikation mit einem Vakzin auf einem der vorgenannten Wege vorliegen und gegebenenfalls hierfür angepasst sein.

Das Lipopeptid oder Lipoprotein nach der Erfindung wird insbesondere synthetisch erhalten. Erfindungsgemäße Lipopeptide könnten auch mit auf dem Fachgebiet allgemein bekannen Verfahren aus einem Mykoplasma-Klon und besonders vorteilhaft aus einem *Mykoplasma-fermentans-*Klon erhalten werden. Die erfindungsgemäßen Lipopeptide werden auch als MALP, nämlich als "macrophage-activating lipopeptides" bezeichnet. Hierzu zählen verschiedene Varianten, von denen MALP-2 ein 2kDa-Lipopeptid gemäß Formel (I) mit Y = GNNDESNISFKEK; R₃, R₄ = H und R₁, R₂ = Palmitoyl(C15) ist (S-(2,3-bispalmitoyloxypropyl)-cysteinyl-GNNDESNISFKEK).

Die Synthese der erfindungsgemäßen Lipopeptide und -proteine kann vorteilhaft nach der Vorschrift in "Synthesis of Nα-Fmoc protected derivates of S-(2,3-dihydroxylpropyl)-cysteine and their application in peptide synthesis", J.W. Metzger, K.-H. Wlesmüller, G. Jung in: Int. J. Peptide Proteine Res. 38, 1991, 545-554" durchgeführt werden. Dort werden allerdings die den natürlichen (beispielsweise aus einem Mykoplasma-Klon enthaltenen) entsprechenden Lipopeptide irrtümlich als S-konfiguriert bezeichnet, obwohl dieselben Verbindungen gemäß der international gültigen Nomenklatur nach Cahn-Ingoid-Prelog als R-konfiguriert bezeichnet werden müssten (siehe z.B. D. Chapman in "Introduction to Lipids", Mc Graw-Hill, London, 1969, S. 67 zu "Phosphoglycerides": Burgos et al. J. Org. Chem. 1987, 52 4973-4977; Morr et al. Eur. J. Immunol. 2002, 32:3337-3347).

in Weiterbildung der Erfindung kann das Lipopeptid oder Lipoprotein in einer Zubereitung mit wenigstens einem weiteren Adjuvans und/oder Antigen vorliegen. Insbesondere kann es mit ein oder mehreren entzündungshemmenden, anti-angiogenen, cytotoxischen oder immunmodulatorischen Stoffen oder Liganden (z.B. Chemokinen, Cytokinen, CD40-Ligand) oder mit Antikörpern gemeinsam verabreicht oder in einer Zubereitung mit diesen gegeben werden.

Das Lipopeptid oder Lipoprotein kann auch, wie oben bereits angesprochen, mit einem physikalischen oder biologischen Träger assoziiert oder verbunden sein. Es kann weiterhin in einer Zubereitung mit weiteren Zusatz- und Hilfsstoffen, insbesondere Konservierungsstoffen oder Stabilisatoren enthalten sein und eingesetzt werden.

Die Erfindung stellt einen großen Fortschritt bei den Bemühungen zur Bereitstellung effektiver Mukosal-Adjuvantien dar. Das Lipopeptid kann im Rahmen der Formel (I) modifiziert und damit in seiner qualitativen und quantitativen Wirksamkeit variiert und an die gewünschte Verwendung angepasst werden. Es ist vergleichsweise kostengünstig herstellbar und für den Menschen nicht toxisch. Vergleichsweise geringe Bemischungsmengen genügen in den meisten Fällen für deutliche Verstärkungseffekte. Die erfindungsgemäßen Lipopeptide sind in ihren chemischen und biochemischen Eigenschaften gut charakterisiert und besonders auf synthetischem Wege genügend rein erhältlich (Mühlradt, P.F., M. Kiess, H. Meyer, R. Sussmuth, G. Jung (1997), Isolation, structure, eludication, and synthesis or a macrophage stimulatory lipopeptide from Muycoplasma fermentans acting at picomolar coticentration"; J. Exp. Med. 185: 1951; and Takeuchl, O., A. Kaufmann, K. Grote, T. Kawai, K. Hoshino, M. Morr, P.F. Mühlradt, and S. Akira (2000); "Cutting edge: Preferentially the R-stereoismoser of the mycoplasmal lipopeptide macrophage-activating lipopeptide-2 activates immune cells through a toil-like receptor 2-and MyD88-dependent signaling pathway"; J. Immunol. 164:554), schwerwiegende Nebenwirkungen sind daher nach derzeitiger Kenntnis nicht zu erwarten.

Ein weiterer Vorteil der Erfindung besteht darin, dass bei Immunisierungen unter Beimischung des erfindungsgemäßen Mukosal-Adjuvans hohe Konzentrationen an IgA in den Schleimhautsekreten behandelter Versuchstiere gefunden wurden. Diese Antikörperspezies ist für den Schutz der Schleimhäute vor Infektionen von besonderer Bedeutung.

Da derzeit überhaupt keine wirksamen Adjuvantien für die intranasale Immunisierung bei humanen Patienten zugelassen sind, stellt die Erfindung einen bedeutenden medizinischen Fortschritt auf diesem Gebiet dar.

Die in dieser Erfindung beschriebenen Lipopeptide oder Lipoproteine der allgemeinen Struktur (I) lassen sich auch generell, d.h. auf anderen Wegen als dem mukosalen, als Adjuvantien verwenden, einschließlich besonderer Applikationen für DNA-Vakzine und Krebsvakzine, Vakzine gegen nicht infektiöse Krankheiten und dergleichen, wobei die vorbekannten MALP-2-Peptide, nämlich S-(2,3-Diacyloxypropyl)cystein-peptide der Sequenz DhcGNNDESNISFKEK, wobei N-terminal die Aminosäuren an Positionen 2 und gegebenenfalls 3 fehlen und/oder C-terminal 1 bis 2 Aminosäuren deletiert sein können, für die üblichen Vakzinierungswege (intramuskulär, subkutan, intradermal, intraperitoneal) ausgenommen werden.

Die erfindungsgemäßen Adjuvantien lassen sich mit den verschiedensten Antigenen zu Impfstoffen kombinieren. Als Antigene können insbesondere Zielantigene für die Prophylaxe und Behandlung von Infektionskrankheiten, Tumoren, Autoimmunkrankheiten, Allergien sowie chronischer oder akuter entzündlicher Krankheiten ausgewählt werden. Die Auswahl kann u.a. aus dem Antigen-Pool von Infektionserregem wie Viren, Bakterien, Parasiten, Rickettsia, Mycoplasma, Pilzen und dergleichen erfolgen. Unter einer Impfung wird auch eine Behandlung mit Antigenen zur Fertilitätskontrolle in menschlichen oder tierischen Populationen verstanden.

Die aus den Beispielen zu entnehmenden vorteilhaften Eigenschaften des erfindungsgemäßen Mukosal-Adjuvans' lassen sich aus keiner Veröffentlichung vor dieser Anmeldung ableiten.

### Methodischer Ansatz für die Experimente/Untersuchungen

Im Vorfeld wurden zunächst in vitro-Screening Studien durchgeführt, um das Potential der untersuchten Lipopeptide bezüglich der Aktivierung antigenpräsentierender Zellen abschätzen zu können. Als Zielzellen wurden knochenmarkstämmige dendritische Zellen verwendet, die aus Vorläufern mit Hilfe von GM-CSF erhalten wurden. Ganz im Gegensatz zu dem, was aufgrund der Aktivität von MALP -2 gegenüber Makrophagen erwartet werden konnte, zeigte MALP-2 nur schwache Aktivität gegenüber primären dendritischen Zellen. Im Vergleich mit den Kontrollproben, die im Beisein von E. coli Lipopolysacchariden (LPS, 10 ng/ml) inkubiert worden waren, wurde eine sehr schwache Aktivierung dendritischer, mit 5 ng/ml MALP-2 behandelter Zellen beobachtet.

Aufgrund dieser Voruntersuchungen konnte für MALP-2 und entsprechende Lipopeptide keine Eignung als Adjuvans erwartet werden, da von Adjuvantien eine gewisse Fähigkeit zur Aktivierung dendritischer Zellen vorausgesetzt wird. Dendritische Zellen sind die Hauptgruppe antigenpräsentierender Zellen überhaupt. Sie spielen bei der primären Immunantwort eine zentrale Rolle, wobei sie (1.) die effektivsten antigenpräsentierenden Zellen darstellen, (2.) die wichtigste Epitopenquelle für spezifische T-Zellen Klone sind, und (3.) die wichtigsten Aktivatoren ruhender T-Zellen sind, welche primäre Immunantworten *in vivo* hervorrufen können. Während mit LPS behandelte dendritische Zellen eine starke Hochregulierung bzw. Vermehrung von CD40 einschließlich CD80 und CD86 zeigen, kann bei MALP-2 behandelten dendritischen Zellen wenn überhaupt, dann nur ein schwacher Effekt bemerkt werden.

Dies war teilweise zu erwarten und in Übereinstimmung mit den an Makrophagen erhaltenen Ergebnissen. Dort war gefunden worden, dass nach einer anfänglichen Hochregullerungsphase bei anhaltender Behandlung mit MALP-2 ein erhöhter Umsatz und verringerte Expression von MHC Klasse II Molekülen, die für die richtige Antigen Präsentation wesentlich sind, auftrat (M. Frisch et al., Eur. J. Immunol. (1996) 26, 1050-1057). Desto überraschender ist die gefundene Wirkung des Adjuvans.

Die Ergebnisse der Vorversuche sprachen daher zunächst stark gegen eine mögliche Aktivität der hier untersuchten Lipopeptide und -proteine als Adjuvantien.

Obwohl *diese in vitro* Studien nahelegten, dass die fraglichen Lipopeptide hier keine Erfolge versprachen, wurden sie als negative Kontrollproben, nämlich als schlecht wirkende Beispiele Makrophagen-aktivierender Substanzen, in *in vivo* Studien der Erfinder mit einbezogen, da die Substanzen aus früheren Versuchen vorhanden waren.

Überraschender Weise, da in völligem Gegensatz zu den *in vitro* Versuchen, wurde gefunden, dass beispielsweise MALP-2 bei Gabe zusammen mit dem Modell, Antigen β-Galaktosidase auf entweder intranasalem (i.n.) oder intraperitonealem (i.p. (Anmerkung: im Tiermodell)) Weg in einer Dosis von nur 0,5 µg pro Tier pro Dosis die β-Galactosidase-spezifischen IgG-Serumtiter um das 675 bis 3560-fache (i.n.) bzw. das 64 bis 128-fache (i.p.) heraufsetzen konnte. Bei Verwendung der erfindungsgemäßen Lipopeptide können bereits nach der Erstimmunisierung annähernd maximale IgG Antworten hervorgerufen werden, und diese IgG Titer entsprechen denen bei Gabe von 10 µg (drelmolarem Überschuss) Cholera-Toxin Untereinheit B (CTB), einem gut charakterisierten mukosalen Adjuvans. Ähnliche Ergebnisse werden bei intradermaler oder subkutaner Verabreichung der erfindungsgemäßen Lipopeptide und -proteine gefunden. Es ist daher davon auszugehen, dass die Gabe der erfindungsgemäßen Lipopeptide und -proteine in wenigstens 3fach geringerer (molarer) Konzentration verglichen mit herkömmlichen Adjuvantien erfolgen, kann und sollte.

Es konnte auch gezeigt werden, dass die Verabreichung der erfindungsgemäßen Lipopeptide auf intranasalem Wege zu einer wirksamen Stimulierung des mukosalen Immunsystems insgesamt führt. So wurden bei Gabe von MALP-2 als Adjuvans zu dem Modell-Antigen bezogen auf das Gesamt-IgA 36 % bzw. 23 % antigenspezifisches IgA in Lungen- und Vaginal-Lavagen gefunden. Dies zeigt, dass die erfindungsgemäßen Lipopeptide nicht nur lokale mukosale Immunantworten hervorrufen können, sondern dass die Verbreitung von IgA-produzierenden Zellen zu anderen entfernten Schleimhautgebieten in einem Maße erfolgt, das zu guten mukosalen Immunantworten führt. Diese Wirkung ist an den mucosalen Verabreichungsweg gebunden.

Die Mitverabreichung der erfindungsgemäßen Lipopeptide zum Antigen rief auch stärkere zelluläre Immunantworten hervor als CTB, sowohl regional in Lymphknoten als auch in der Milz (p < 0,05). Die Analyse β-Galactosidase-spezifischer lgG-lsotypen und die Profile der durch *in vitro* stimulierte Zellen sekretierten Zytokine zeigten, dass die Mitverabreichung der Lipopeptide ein dominantes Th2-Antwortmuster auslöste. Die Ergebnisse der Untersuchungen belegen daher, dass die erfindungsgemäßen Lipopeptide wirksame Adjuvantien für die mukosale Verabreichung von Antigenen in Vakzinen darstellen.

Ein wichtiger Aspekt, der hier hervorgehoben werden soll, ist, dass eine inkrementelle Erhöhung der Lipopeptid-Dosis (von den hier genannten optimalen Dosen ausgehend) zu einer stetigen Verringerung der Immunantwort führt. Dieser gegenläufige Effekt ist unerwartet und war aus dem Stand der Technik nicht herzuleiten.

### BEISPIELTEIL

### Allgemeines

Die in den Beispielen dargestellten Untersuchungen wurden vorrangig mit einem synthetischen, einem aus Mycoplasma stammenden Lipopeptid weitgehend entsprechenden MALP-2 als Mukosal-Adjuvans zusammen mit β-Galaktosidase als Model-Antigen durchgeführt. Dieses spezielle Beispiel-Lipapeptid wurde auf Grund seiner vorher bestimmten intrinsischen Eigenschaften (biochemische Eigenschaften, Makrophagen-stimulierende Aktivität) ausgewählt. Sofern nichts anderes angegeben, ist das in den Beispielen verwendete synthetische Lipopeptid S-[2,3-bispalmitoyloxypropyl]cysteinyl-GNNDESNISFKEK im folgenden stets einfach als "MALP-2" bezeichnet.

Die Dosen für MALP-2 wurden zunächst in Vorstudien eingegrenzt, in denen MALP-2 subkutan., intradermal, intranasal oder intraperitoneal an Mäuse verabreicht wurde. In allen Protokollen führte die gleichzeitige bzw. zusammengehörige Verabreichung von MALP-2 mit β-Galaktosidase zu einem signifikanten Anstieg der Produktion β-Galactosidase-spezifischer Antikörper. Die induzierten Immunantworten, die in Anwesenheit von MALP-2 nach Impfung auf intranalalem oder intraperitonealem Wege erhalten wurden, wurden dann analysiert und mit den In Vergleichsversuchen mit CTB als Adjuvans erhaltenen verglichen.

Die Beispiele ergeben, dass die Verwendung von MALP-2 in einer Dosis von nur 0,5 µg pro Verabreichung zu einer signifikanten Erhöhung sowohl der humoralen als auch der zellulären β-Galactosidase-spezifischen Antworten führte. Bereits intraperitoneale Verabreichung führte zu einer verbesserten Immunantwort, die intranasale Route erwies sich jedoch als noch weit effektiver. Im Gegensatz dazu war ein dreifacher molarer Überschuss an CTB erforderlich um vergleichbare systemische oder mukosale humorale Antworten zu erhalten. Bezüglich der zellulären Antworten zeigten Milzzellen von mit MALP-2 immunisierten Mäusen eine deutlich höhere Proliferation (p < 0,05) als solche aus Tieren, die mit dem dreifachen molaren Überschuss an CTB vakziniert worden waren.

Die mit MALP-2 als Adjuvans auf intranasalem Wege erhaltene Primärantwort wurde bezüglich der Kinetik der β-Galactosidase-spezifischen Antikörper-Antwort durch die Anwesenheit hoher Antikörpertiter charakterisiert, die schon nach der Erstimmunisierung fast das Maximalplateau erreichten. Humorale und zelluläre Antworten fielen nach i.n.-Vakzinierung stärker aus, was auf eine differenzierte lokale Wirkung von MALP-2 hinweist, die entweder auf seine Bioverfügbarkeit oder die räumliche Verteilung der spezifischen Rezeptoren in den Zielzellen zurückgeführt werden könnte. Wie bereits beschrieben wurden auch in durch die Vakzinierungsroute nicht direkt erreichbaren Schleimhautgeweben deutliche Immunantworten gefunden, nämlich β-Galactosidase-spezifisches IgA in Lungen und Vaginal-Lavagen.

Die Untersuchungen im Rahmen der Erfindung haben daher ergeben, dass die erfindungsgemäßen Lipopeptide neue und potente Mukosal-Adjuvantien sind, wobei das Erfordernis, das Ziel-Antigen mit dem aktiven Lipopeptid zu konjugieren, im Gegensatz zu anderen Lipopeptiden entfällt.

Es wurden über den gesamten Verlauf der Experimente bei den geimpften Tieren keine schwerwiegenden Nebenwirkungen oder Zeichen akuter oder chronischer Toxizität beobachtet. Durch die relativ kurze Peptid-Einhait innerhalb der erfindungsgemäßen Lipopeptide besteht eine relativ geringe Immunogenizität. was die Gefahr, dass Immunantworten gegen die Lipopeptide selbst auftreten, minimiert, Dies stellt einen deutlichen Vorteil gegenüber Proteinen als Adjuvantien dar. Es konnten nach intranasaler Verabreichung von MALP-2 als Adjuvans keine anti-MALP-2 Antikörper detektlert werden. Dies stellt einen deutlichen Vorteil gegenüber Proteinen als Adjuvantien dar, da eine Immunantwort gegen das Adjuvans selbst eine spätere Immunantwort gegen einen anderen Impfstoff, der mit demselben Adjuvans verabreicht wurde, beeinträchtigen kann. Die erfindungsgemäßen Lipopeptide können in Impfstoffen gegen verschiedenste Pathogene sowie in Krebsvakzinen oder Vakzinen zur Fertilitätskontrolle eingesetzt werden. Weitere Vorteile der Erfindung liegen in der guten Haltbarkeit der Lipopeptide bei Lagerung, größerer Reinheit der synthetisch herstellbaren Lipopeptide und überschaubarem chemischem und biochemischem Verhalten.

### Legenden der Abbildungen:

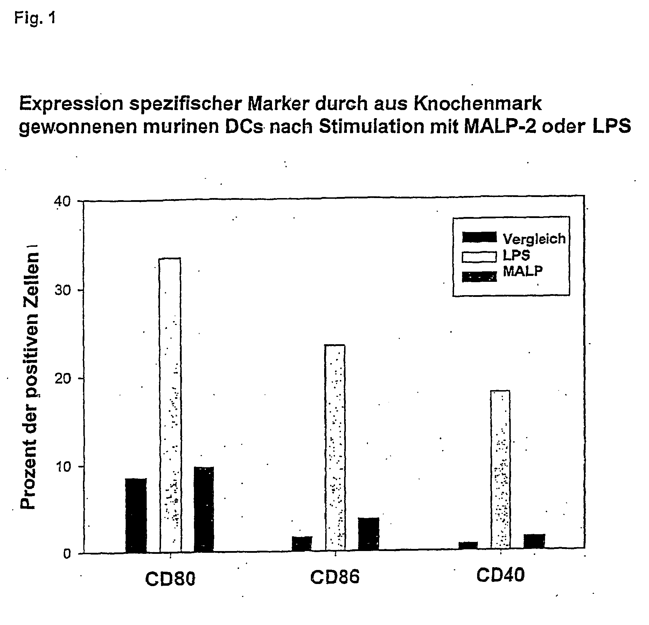

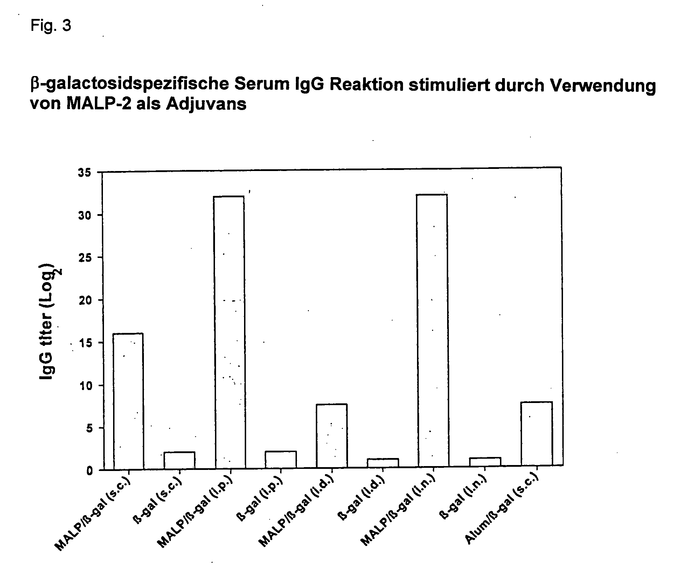

Fig. 1. *In vitro* Untersuchungen mit primären dendritischen Zellen: Primäre dendritische Zellen aus Knochenmark von BALB/c-Mäusen wurden unter Verwendung der Rekombinante GM-CSF (5 x 10⁴ U/ml) durch *in vitro*-Reifung von Vorläufern gewonnen. Die reifen dendritischen Zellen wurden mit 10 ng/ml E. coli Lipopolysaccharid (LPS) beziehungsweise 5 ng/ml MALP2 stimuliert. Anschließend wurden die Zellen mit CD11c-spezifischen Antikörpern (Dendritenzellmarkern) in Kombination mit Anti-CD40 oder Anti-CD80 oder Anti-CD86 doppelt markiert. Die Expression von CD40, CD80 beziehungsweise CD86 in den CD11c-markierten Zellen wurde mit Hilfe der Durchflusszytometrie analysiert. Die Ergebnisse sind in Prozent positiver Zellen bezüglich der gesamten CD11c-positiven Population ausgedrückt.

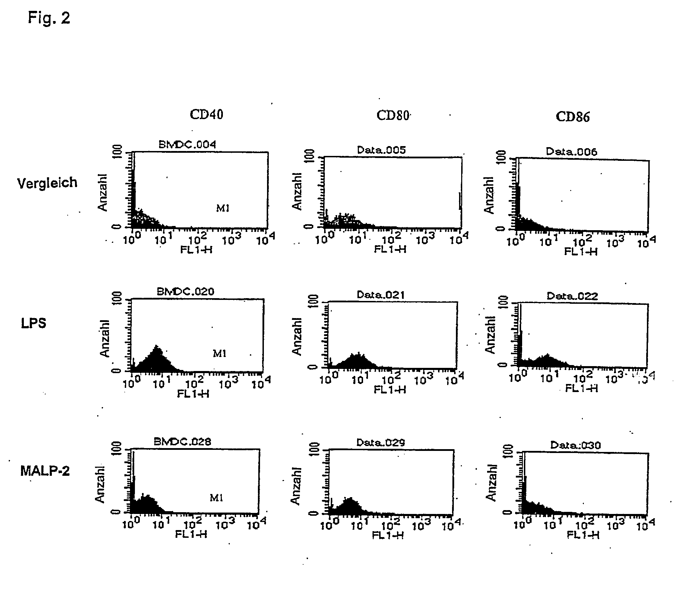

Fig. 2. Analyse dendritischer Zellen nach Behandlung mit MAPL-2 mit Hilfe der Durchflusszytometrie (FACScan):
   Primäre dendritische Zellen aus Knochenmark von BALB/c-Mäusen wurden unter Verwendung der Rekombinante GM-CSF (5 x 10⁴ U/ml) durch *in vitro*-Reifung von Vorläufern gewonnen. Die reifen dendritischen Zellen wurden mit 10 ng/ml E. coli Lipopolysaccharid (LPS) beziehungsweise 5 ng/ml MALP2 stimuliert. Anschließend wurden die Zellen mit CD11c-spezifischen Antikörpern (Dendritenzellenmarkern) in Kombination mit Anti-CD40 oder Anti-CD80 oder Anti-CD86 doppelt markiert und die Zellen wurden mittels Durchflusszytometrie analysiert. Die Gates wurden auf der Grundlage einer Markierung mit nichtverwandten Kontrollantikörper-Isotopen gesetzt
Fig. 3. Humoralantworten, angeregt nach einer Impfung mit MAPL-2 als Adjuvans:
   Mäuse wurden über subkutane (s.c.), intraperitoneale (i.p.), intradermale (i.d.) und intranasale (i.n.) Routen mit entweder reiner β-Galactosidase (40 µg) oder β-Galactosidase gemischt mit MALP-2 (0.5 µg) an den Tagen 0, 7 und 14 immunisiert. Am Tag 28 nach der primären Immunisierung wurden Serumproben entnommen und der Titer der β-Galactosidase-spezifischen Antikörper wurde mittels ELISA bestimmt. Die Ergebnisse sind als der reziproke log₂ des geometrisch mittleren Endpunkttiters dargestellt. Zur Kontrolle bezogen wir eine Gruppe ein, in welcher die Tiere über die Route mit β-Galactosidase unter Verwendung von Aluminiumhydroxid als Adjuvans immunisiert wurden.

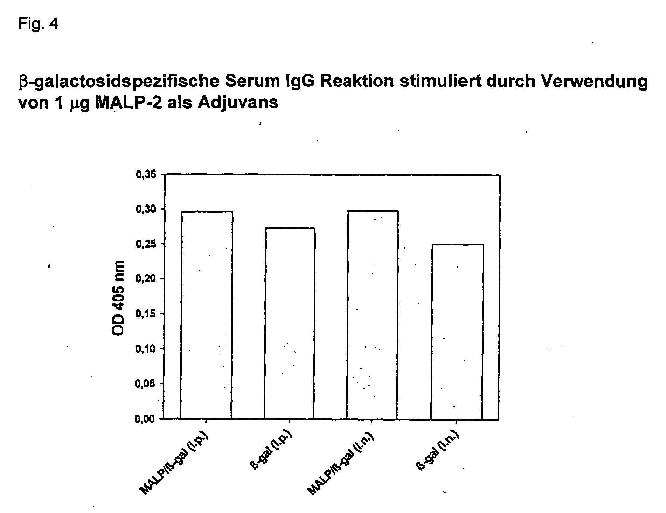

Fig. 4. Humoralantworten, angeregt nach der Impfung unter Verwendung von MALP-2 als Adjuvans in einer Dosis von 1 µg pro Tier und Immunisierung:
   Mäuse wurden über die intraperitoneale (i.p.) und intranasale (i.n.) Route mit entweder reiner β-Galactosidasa (40 µg/Dosis) oder β-Galactosidase gemischt mit MALP-2 (1 µg/Dosis) an den Tagen 0, 7 und 14 immunisiert. Am Tag 28 nach der primären Immunisierung wurden Serumproben entnommen und der Titer der β-Galactosidase-spezifischen Antikörper wurde mittels ELISA bestimmt. Die Ergebnisse sind als Absorptionswerte (OD 405 mm) dargestellt.

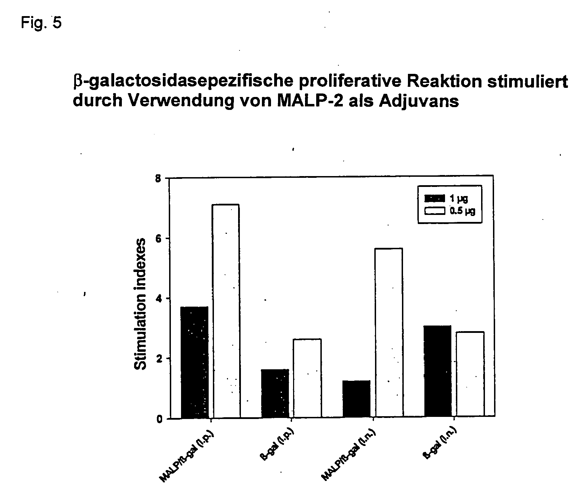

Fig. 5. Zettreaktionen, angeregt nach einer Impfung unter Verwendung unterschiedlicher Konzentrationen von MALP-2 als Adjuvans. β-Galactasidase-spezifische T-Zell-Ptoilferatorisantworten von Mäusemilzzellen immunisiert über die Routen i.p. oder i.n. mit entweder reiner β-Galactosidase (40 µg/Dosis) oder β-Galactosidase gemischt mit MAPL-2 (1 oder 0.5 µg/Dosis) an den Tagen 0, 7 und 14. Am 28. Tag nach der primären Immunisierung wurden die Tiere getötet und die Milzzellen wurden in vitro vier Tage im Beisein von 20 µg/ml löslicher ß-Galactosidase re-stimuliert. Die Ergebnisse sind als Stimulationsindizes (cpm Proben/cpm in nichtstimulierten Kontrollzellen) dargestellt.

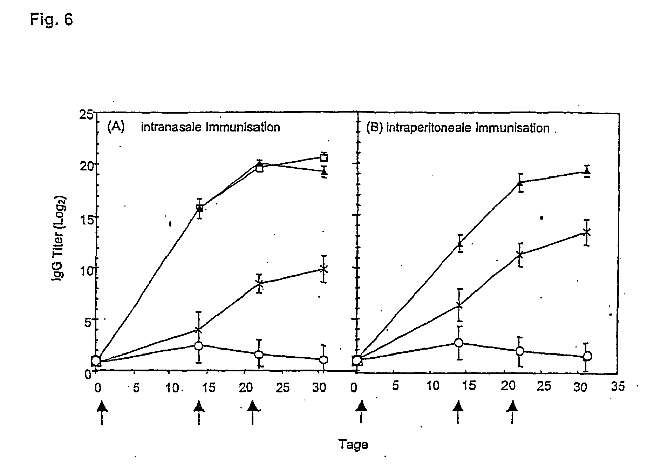

Fig. 6. Kinetik von β-gal-spezifischen IgG-Antworten in Seren geimpfter Mäuse.:
   Gruppen von Tieren (n=5) wurden entweder i.n. (A) oder i.p. (B) mit 50 µg β-gal (), - β-gal plus 10 µg CTB (), β-gal plus 0.5 µg MALP-2 () oder reiner Pufferlösung () geimpft. Die Tage der Impfungen (Tag 0, 14 und 21) sind mit den Pfeilen markiert. Die Ergebnisse sind als der reziproke log₂ des geometrischen Endpunkttiters dargestellt, die SEM (mittlere Standardabweichung) ist mittels senkrechter Linien angegeben.
Fig. 7. β-Galactosidase-spezifischer IgA in Lungen- und Vaginalausspülungen von i.n.-geimpften Mäusen:
   Die Ergebnisse sind als Prozent β-Galactosldase-spezifischer IgA bezüglich des insgesamt vorhandenen IgA dargestellt. Die SEM ist durch senkrechte Linien angegeben.
Fig. 8. Bestimmung der Serum-IgE-Niveaus nach Immunisierung unter Verwendung von MALP-2 als Adjuvans:
   Mäuse wurden Intraperitoneal (i.p.) und intranasal (i.n.) entweder mit reiner β-Galactosidase (40 µg), ausschließlich MALP-2 oder β-Galactosidase gemischt mit MALP-2 (0.5 µg) an den Tagen 0, 7 und 14 geimpft. Am 28. Tag nach der Erstimmunisierung wurden Serumproben genommen und die Niveaus von IgE wurden mittels eines Capture-ELISAs bestimmt. Die Ergebnisse sind als IgE-Konzentrationen (ng/ml) dargestellt.

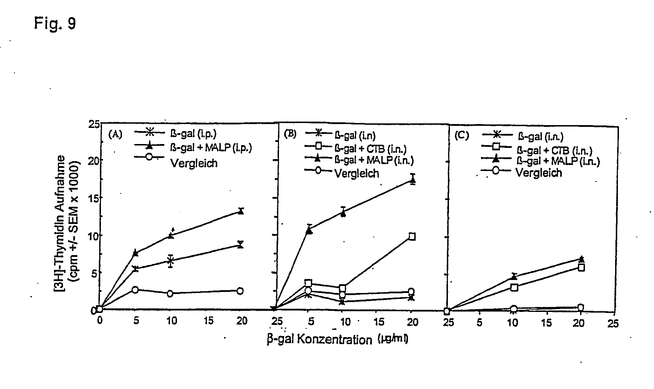

Fig. 9. β-Galactosidase-speziflsche T-Zell-Proliferationsantworten der Milz (A und B) und regionaler Lymphknotenzellen (C) von i.p. oder i.n. geimpften Mäusen:
   Die Zellen wurden in vitro vier Tage lang mit unterschiedlichen Konzentrationen löslicher β-Galactosidase re-stimuliert. Die Ergebnisse sind als mittlerer cpm-Wert subtrahiert von den Hintergrundwerten nichtstimulierter Zellen aus Dreifachgruppen dargestellt. Die SEM ist durch senkrechte Linien dargestellt.

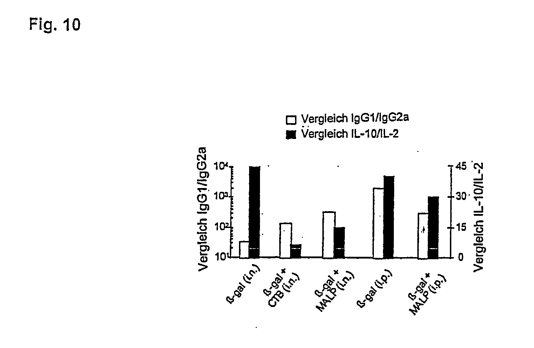

Fig. 10. Im geimpften Mäusen stimulierte Th-Profile:
   Serum-β-Gaiactosidase-spezifische lgG-lsotypen und -Zytokine, ausgeschieden von in vitro stimulierten Milzzellen, wurden bei den geimpften Mäusen mittels ELISA bestimmt. Die Ergebnisse sind als Konzentrationsverhältnisse von lgG1/lgG2a und IL-10/IL-2 (die am häufigsten gefundenen Zytokine) dargestellt.

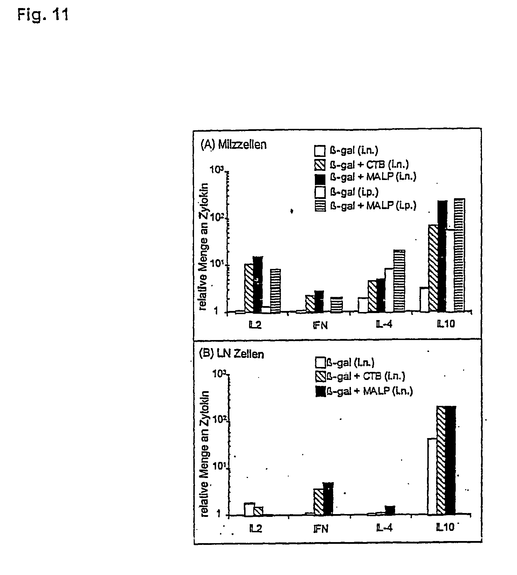

Fig. 11. Zytokine ausgeschieden von in vitro stimulierten Zellen geimpfter Mäuse:
   Die Zytoklnproduktion wurde mittels ELISA im Flüssigkeitsüberstand von Zellen, weiche 48 (IL-2) beziehungsweise 96 Stunden (IFNγ, IL-4 und IL-10) lang im Beisein von β-gal (20 µg/ml) kultiviert wurden, gemessen. Die Ergebnisse sind als das Verhältnis zwischen den in den geimpften Gruppen vorgefundenen Zytokinmengen im Vergleich zu den nicht-geimpften Kontrollmäusen dargestellt.

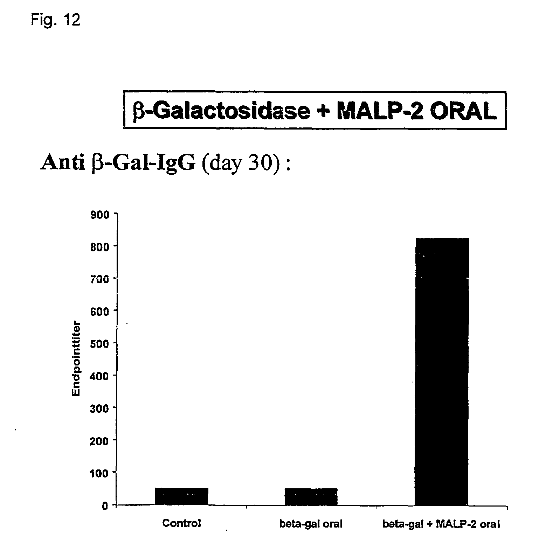

Fig. 12: Humoralantworten stimuliert nach Vakzinierung mit MALP-2 als Adjuvans in einer Dosis von 2 µg pro Tier pro Immunisierung. Die Mäuse wurden entweder mit β-Galactosidase alleine (100 µg/Dosis) oder mit β-Galactosidase gemischt mit MALP-2 (2 µg/Dosis) an den Tagen 0, 7, 14 und 31 oral immunisiert. Am Tag 45 nach der Erstimmunisierung wurden Serumproben genommen und die Titer der β-Galactosidase-spezifischen Antikörper wurden mit ELISA bestimmt.

### Beispiel 1: In vitro-Stimulierung von primären aus dem Knochenmark gewonnenen dendritischen Nagetierzellen mittels MALP-2.

*Versuchsprotokoll:* Kulturen primärer aus dem Knochenmark gewonnener dendritischer Zellen wurden von BALB/c-Mäusen nach in vitro-Reifung von Vorläufern im Beisein der Rekombinante GM-CSF (5 x 10⁴ U/ml) nach den üblichen Verfahren erhalten. Die reifen dendritischen Zellen wurden mit E. coli Lipopolysaccharid (LPS) oder 5 ng/ml MALP2 stimuliert. Nach 12 beziehungsweise 24 Stunden Stimulierung wurden die Zellen mit Hilfe der Durchflusszytometrie analysiert, um die Expression von Oberflächenmarkern, die für die Fähigkeit zur Antikörperpräsentation von Bedeutung sind, festzustellen.

Um Verbindungen zu bestimmen, welche bei *in-vivo*-Anwendungen auf dem Gebiet der Vakzinierungen ein Potential als Adjuvans besitzen, wurde eine erste *in-vitro-*Untersuchung mit primären Kulturen aus Knochenmark stammender dendritischer Zellen durchgeführt. Es wurden dendritische Zellen ausgewählt, da sie die effektivsten Antigen-präsentierenden Zellen sind und bei der primären Immunantwort eine Schlüsselrolle spielen. Tatsächlich sind sie der einzige Zelltyp, der *in vivo* in der Lage ist, ruhende T-Zellen zur Initlierung einer primären Immunantwort zu aktivieren. Demzufolge wurden dendritische Zellen mit den untersuchten Einheiten oder LPS, was zur Kontrolle diente, behandelt. Es wurden zu unterschiedlichen Zeitpunkten Proben entnommen, mit fluoreszenz-markierten Antikörpern, die spezifisch sind für zelluläre Marker, welche für die antigenpräsentierenden Fähigkeiten der dendritischen Zellen entscheidend sind, markiert und mittels Durchflusszytometrie analysiert. Die vorgefundenen Ergebnisse (Fig. 1 und 2) zeigen, dass im Unterschied zur positiven Kontrollgruppe die Expression von CD40 und dem ko-stimullerenden Molekül CD86 in den mit MALP-2 behandelten dendritischen Zellen nicht erhöht war. Die Wirkung auf die Expression des co-stimulierenden Moleküls CD80 war gering, falls überhaupt vorhanden. Costimulierende Moleküle senden Signale, welche zusätzlich zur Präsentation der beteiligten Epitope innerhalb des Kontextes der MHC-Moleküle für die wirksame Aktivierung der T-Zellen wesentlich sind. Es wurde früher schon berichtet, dass die Wirkung bewährter Schleimhautadjuvantien (Mukosal-Adjuvantien) wie z.B. Choleratoxin mit einer selektiven Verstärkung der Expression co-stimulierender Moleküle verbunden ist. Demzufolge deuten die *in vitro* gefundenen Ergebnisse stark darauf hin, dass MALP-2 kein oder geringes Potential als Schleimhautadjuvans aufweist.

### Beispiel 2: Humoralantworten, angeregt nach einer Impfung über unterschiedliche Wege unter Verwendung von MALP-2 In unterschiedlichen Konzentrationen als Adjuvans.

*Versuchsprotokoll:* Sechs bis acht Wochen alte weibliche BALB/c- (H-2d) Mäuse wurden von der Harlan Winkelmann GmbH (Borchen, Deutschland) gekauft und gemäß den örtlichen und EU-Richtlinien behandelt. Gruppen von jeweils 5 Mäusen wurden am Tag 1, 7 und 14 entweder mit 40 µg reiner ß-Galactosidase (Boehringer, Mannheim, Deutschland), oder unter Zumischung von 1 beziehungsweise 0,5 µg von synthetischem MALP-2 geimpft. Für die intranasale Verabreichung (i.n.) wurden 10 µl in jedes Nasenloch appliziert, während bei der Injektion i.p., s.c. und i.d. ß-Galactosidase mit beziehungsweise ohne MALP-2 in 400, 100 beziehungsweise 100 µl PBS re-suspendiert wurde. 28 Tage nach der Erstimpfung wurden Serumproben entnommen und bei -20°C bis zur Bestimmung der β-gal-spezifischen Antikörper aufbewahrt. Nune-Immuno MaxiSorp-Testplatten mit 96 Mulden (Nunc, Roskilde, Dänemark) wurden mit 100 µl β-Galactosidase (Boehringer, Mannhelm, Deutschland) mit 5 µg/ml in 0.05 M Carbonatpuffer (pH 8.2) pro Mulde beschichtet. Serumverdünnungen mit 1 % BSA und 0,05 % Tween-20 in PBS wurden hinzugefügt (100 µg/Mulde), und die Platten wurden bel 37°C 24 Stunden lang inkubiert. Nach dem Spülen wurde biotinyliertes γ-kettenspezifisches Ziegen-Antimaus-lgG (Sigma Chemie, Deisenhofen, Deutschland) hinzugefügt und die Platten wurden eine weitere Stunde lang bei 37°C inkubiert. Nach vierfachem Spülen wurden 100 µl peroxidase-konjugiertes Streptavidin (Pharmingen) zu den Zellen hinzugegeben und die Platten wurden 30 Minuten lang bei 37°C inkubiert. Nach vierfachem Spülen wurden die Reaktionen mittels ABTS in 0,1 M Zitratphosphatpuffer (pH 4,35), welcher 0,01 % H₂O₂ enthielt, entwickelt. Die Ergebnisse wurden entweder nach der Absorption bei 405 nm oder den Endpunkttitem (reziproker log₂ der letzten Verdünnung, welche nach 30 Minuten Inkubation bei 405 nm eine optische Dichte von 0,1 Einheiten über den Werten der negativen Kontrollgruppe ergab) dargestellt.

Trotz der enttäuschenden Ergebnisse, welche bei der *In vitro* Untersuchung von primären dendritischen Zellen unter Verwendung von MALP-2 festgestellt wurden, beschlossen wir, bei der sekundären in vivo Untersuchung Gruppen von Tieren einzubeziehen, die unter Verwendung von MALP-2 als Adjuvans geimpft worden waren. Demzufolge wurden die Mäuse entweder mit dem reinen Modellantigen ß-Galactosidase oder mit dem Antigen plus MALP-2 i.p., s.c., i.d. und i.n. geimpft. Im Gegensatz zu den Erwartungen stellte man eine starke Adjuvanswirkung fest, wenn das Antigen mit 0,5 µg MALP-2 gemischt war, und zwar unabhängig von der Art und Weise der Applikation (Fig. 3). Die stärksten Reaktionen wurden bei den Impfungen i.p, und i.n. festgestellt. Die erhaltenen Antworten waren jedoch immer wenigstens genauso stark (i.d.) oder stärker als bei der Verwendung von Aluminiumhydroxid als Standardadjuvans bei s.c. Injektion (Fig. 3).

Da bei vorangegangenen Untersuchungen unter Verwendung konventioneller Lipopeptide wesentlich höhere Konzentrationen des als Adjuvans verwendeten Bestandteils gegeben wurden, wurde die Wirkung höherer Dosen von MALP-2 zu untersuchen. Zu diesem Zweck wurden Tiere mit 1 µg MALP-2 als Adjuvans über die zwei wirksamsten Immunisationswege (i.p. und i.n.) mit β-Galactosidase geimpft. Im Gegensatz zu den Erwartungen führte die Erhöhung der Dosis von MALP-2 zu einer Aufhebung der Adjuvanswirkung (Fig. 4). Dies zeigte, dass wir bei einer Anwendung der Standardkonzentrationen, welche in der Literatur für andere Lipopeptide angegeben sind, bei den *in vivo* Untersuchungen von MALP-2 nicht in der Lage gewesen wären, eine Adjuvanswirkung auf der Ebene der Humoralantworten festzustellen.

### Beispiel 3: Humoral Immunantworten, angeregt nach Impfung über verschiedene Wege unter Verwendung von MALP-2 als Adjuvans in unterschiedlichen Konzentrationen.

*Versuchsprotokoll:* Sechs bis acht Wochen alte weibliche BALB/c- (H-2d) Mäuse wurden von der Harlan Winkelmann GmbH (Borchen, Deutschland) gekauft und gemäß den örtlichen und EU-Richtlinien behandelt. Gruppen von jeweils 5 Mäusen wurden am Tag 1, 7 und 14 entweder mit 40 µg reiner β-Galactosidase (Boehringer, Mannheim, Deutschland), oder unter Zumischung von 1 beziehungsweise 0,5 µg synthetischem MALP-2 geimpft. Für die intranasale Verabreichung (i.n.) wurden 10 µl in jedes Nasenloch appliziert, während bei der Injektion i.p. β-Galactosidase mit beziehungsweise ohne MALP-2 in 400 µl PBS re-suspendiert wurde. Die Milzen wurden entfernt und für die Bestimmung der zellulären Immunreaktionen zusammengenommen (gepoolt). Die Zellen wurden in RPMI 1640 ergänzt mit 10 % Kalbsfötenserum, 100 U/ml Penizillin, 50 µg/ml Streptomycin, 5 x 10⁻⁵ M 2-Mercaptoethanol und 1 mM L-Glutamin (GIBCO BRL, Karisruhe, Deutschland) bei 37°C in einer feuchten Atmosphäre mit 5 % CO₂ kultiviert. Die Milzzellensuspensionen wurden auf 5 x 10⁶ Zellen/ml im vollständigen Medium eingestellt. Sie wurden zu 100 µl/Mulde in einer flachbödigen Mikrotiterplatte (Nunc) mit 96 Mulden eingebracht und diese Platten wurden 4 Tage lang unter Beifügung von 20 µg/ml löslicher β-Galactosidase inkubiert. Während der letzten 18 Stunden der Kultivierung wurden jeder Mulde 1 µCi [³H]Thymidin (Amersham International, Freiburg, Deutschland) zugefügt. Die Zellen wurden dann mit einem Zellenernter (Inotech, Wohlen, Schweiz) auf Filterpapier geerntet (Filtermat A; Wallac, Freiburg, Deutschland) und die Menge des in die DNS der vermehrten Zellen eingebetteten [³H]Thymidins wurde mit Hilfe eines γ-Scintillationszählers (Wallac 1450, Micro-Trilux) bestimmt. Die Ergebnisse sind als das arithmetische Mittel der [³H]Thymidinaufnahme In cpm dargestellt. Die Ergebnisse sind als Stimulationsindices (SI, cpm Proben/cpm in nicht-stimulierten Kontrollzellen) dargestellt.

Unter Berücksichtigung der überraschenden Verringerung der Adjuvanswirkung auf humoraler Ebene, die bei der Verwendung von MALP-2 in höheren Dosierungen beobachtet wurde, wurde beschlossen, festzustellen, ob eine ähnliche Wirkung auf der Ebene der zellulären Immunantworten beobachtet werden kann. Demzufolge wurden Mäuse mit entweder reiner ß-Galactosidase oder β-Galactosidase gemischt mit 1 µg MALP-2 immunisiert. Achtundzwanzig Tage nach der Immunisierung wurden die Milzen gereinigt, in vitro mit 20 µg/ml β-Galactosidase re-stimuliert und ihre Vermehrungsfähigkeit wurde mit Hilfe der Messung der Einbettung von [^{3H}]Thymidin in ihre DNS mittels eines γ-Scintillationszählers bestimmt. Die erzielten Ergebnisse (Fig. 5) bestätigten, dass die Verwendung von MALP-2 in höheren Dosierungen nicht nur dazu führt, dass keine Humoralantworten mehr festgesellt werden können, sondern auch die über die Zellen vermittelte Immunisierung wird verringert.

### Beispiel 4: Gemeinsame intranasale und intraperitoneale Verabreichung von MALP-2 mit einem löslichen Antigen stimuliert wirksame systemische Humoralantworten.

*Versuchsprotokoll:* Sechs bis acht Wochen alte weibliche BALB/c- (H-2d) Mäuse wurden von der Harlan Winkelmann GmbH (Borchen, Deutschland) gekauft und gemäß den örtlichen und EU-Richflinien behandelt. Gruppen von jeweils 5 Mäusen wurden am Tag 1, 14 und 21 entweder mit 40 µg reiner β-Galactosidase (Boehringer, Mannheim, Deutschland) unter Zumischung von 0.5 µg synthetischer MALP-2 beziehungsweise 10 µg Choleratoxin B Untereinheit (CTB; ICN Biomedicals Inc., Ohio) als Standardadjuvans geimpft, Für die intranasale Verabreichung (i.n.) wurden 10 µl in jedes Nasenloch appliziert, während bei der Injektion i.p. β-Galactosidase mit beziehungsweise ohne MALP-2 in 400, 100 beziehungsweise 100 µl PBS re-suspendiert wurde. Serumproben wurden zu verschiedenen Zeitpunkten entnommen (Tag 0, 13, 20 und 30) und bei -20°C bis zur Bestimmung der β-Galactosidase-spezifischen Antikörper aufbewahrt. Nunc-Immuno MaxiSorp-Testpiatten mit 96 Mulden (Nunc, Roskilde, Dänemark) wurden mit 100 µl β-gal (Boehringer, Mannheim, Deutschland) mit 5 µg/ml in 0.05 M Karbonatpuffer (pH 8.2) pro Mulde beschichtet. Serielle zweifache Verdünnungen der Seren oder Auswaschungen in PBS mit 1 % BSA und 0,05 % Tween-20 wurden hinzugefügt (100 µl/Mulde) und die Platten wurden bei 37°C 24 Stunden lang inkubiert. Nach dem Spülen wurde biotinyliertes γ-kettenspezifisches Ziegen-Antimaus-IgG (Sigma Chemie, Deisenhofen, Deutschland) hinzugefügt und die Platten wurden eine weitere Stunde lang bei 37°C inkubiert. Nach vierfachem Spülen wurden 100 µl peroxidase-konjugiertes Streptavidin (Pharmingen) zu den Zellen hinzugegeben und die Platten wurden 30 Minuten lang bei 37°C inkubiert. Nach vierfachem Spülen wurden die Reaktionen mittels ABTS In 0,1 M Zitratphosphatpuffer (pH 4,35), welcher 0,01 % H₂O₂ enthielt, entwickelt Die Ergebnisse wurden als der reziproke log₂ der letzten Verdünnung, welche nach 30-minütiger Inkubation eine optimale Dichte bei 405 nm von 0,1 Einheiten im Vergleich zu den Werten der negativen Kontrollgruppe ergab, dargestellt. Aufgrund der ermutigenden Ergebnisse der Vorstudien beschlossen wir, die bei der Verwendung von MALP-2 als Adjuvans über die beiden wirksamsten Immunisierungswege, nämlich i.p. und i.n., festgestellten Immunreaktionen detailliert zu analysieren und sie mit denen eines bewährten Schleimhautadjuvans zu vergleichen. Demzufolge wurde die Fähigkeit von MALP-2, wirksame Humoraliimmunreaktion hervorzurufen bewertet, indem die Serumtiter von ß-Galactosidase-spezifischen Antikörpern bei geimpften Mäusen bestimmt wurden. Wie in Fig. 6A dargestellt, ergab die Administration von reiner ß-Galactosidase (50 µg/Dosis) die Induktion sehr niedriger Antikörpertiter, sogar nach einem zweiten Boosting (Endpunkttiter ca. 1000). Im Vergleich dazu induzierte die Administration von β-Galactosidaso unter Verwendung von MALP-2 i.n. schon bei einer einfachen Dosis zu Induktion sehr hoher Titer (> 60.000) von spezifischem IgG bei allen Mäusen, und am Ende des Immunisierungsprotokolls lagen die Titer über 500.000 (Fig. 6). Die Kinetik und die Gesamtwirksamkeit der Antikörperreaktionen, die mit 0,5 µg MALP-2 erreicht wurden, waren denen, die mit der Verabreichung von β-Galactosidase mit 10 µg CTB, einem bewährten Schleimhautadjuvans, welches als positive Kontrollsubstanz verwendet wurde, erreicht wurden, sehr ähnlich.

Eine deutliche Adjuvanswirkung wurde auch beobachtet, wenn MALP-2 i.p. appliziert wurde. Insbesondere die Co-Injektion von MALP-2 führte zu einer 100-fachen Steigerung der β-Galactosidase-spezifischen IgG-Titer im Vergleich zu den Titern bei Tieren, welche mit reiner β-Galactosidase immunisiert worden waren (Fig. 6B). Dieser Unterschied war schon nach der ersten immunisierung festzustellen und wurde nach Booster-Injektionen aufrechterhalten. Am 31. Tag wurden bei Tieren, die entweder i.n. oder i.p. immunisiert worden waren, ähnliche Antikörpertiter festgestellt. Allerdings waren die Primärreaktionen nach der MALP-2-Co-Injektion nach der i.n. Impfung stärker ausgeprägt.

### Beispiel 5: Intranasale Co-Administration von MALP-2 mit einem löslichen Antigen stimuliert wirksame Schleimhautantikörperantworten.

*Versuchsprotokoll:* Am 31. Tag wurden die Mäuse getötet und es wurden die endgültigen Proben entnommen. Es wurden Vaginal- und Lungenausspülungen gewonnen, indem die Organe mit 1 ml PBS, ergänzt durch 50 mM EDTA, 0,1 % BSA und 10 mM PMSF gespült wurden. Die Ausspülungen wurden anschließend zentrifugiert, um Gewebstrümmer zu entfernen (10 min bei 3000 x g) und die verbleibende Flüssigkeit wurde bei -20°C aufbewahrt. Um die Konzentration des Gesamt-IgA in der Spülflüssigkeit aus Lunge und Vagina zu bestimmen, wurden serielle Verdünnungen der entsprechenden Proben in Mikrotiterplatten inkubiert, wobei diese Platten zuvor mit Ziegen-Nitmaus-IgA (Sigma Chemie) als Einfangantikörper (100 µl/Mulde) überzogen wurden. Es wurden serielle Verdünnungen gereinigter Maus-lgA (Sigma Chemie) zur Erzeugung einer Standardkurve verwendet.

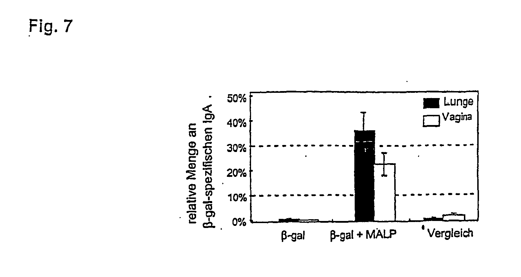

Um die Fähigkeit von MALP-2 zur Stimulierung von Mukosal-Antworten gegen Antigene, die i.n. co-administriert wurden, zu untersuchen, wurde die Produktion von β-Galactosidase-spezifischem IgA in Lungen- und Vaginalspülflüssigkeit immunisierter Tiere. Während nach der i.n. Impfung mit reiner β-Galactosidase keine Produktion β-Galactosidase-spezlflscher IgA in der Lungenspülflüssigkeit in nachweisbarer Menge erfolgte, wurde bei den mit β-Galactosidase und MALP-2 immunisierten Tieren ein signifikanter Anstieg der Niveaus antigenspezifischer IgA festgestellt (Fig. 7). Die Co-Administration von MALP-2 führte zur Stimulierung einer wirksamen IgA-Produktion auch in entfernt liegenden Schleimhäuten, wie dies durch die Anwesenheit signifikanter Konzentrationen von β-Galactosidase-spezifischem IgA in der Vaginalspülflüssigkeit nachgewiesen wird (Fig. 7). Es wurden keine statistisch signifikanten Unterschiede in den Niveaus der β-Galactosidasespezifischen Antikörper in den Schleimhäuten zwischen Tieren, die mit 0.5 µg MALP-2 oder 10 µg CTB immunisiert worden waren, festgestellt

### Beispiel 6: Die Immunisierung unter Verwendung von MALP-2 als Adjuvans führt nicht zu mehr Serum-lgE.

*Versuchsprotokoll,* Zur Bestimmung der Konzentration des Gesamt-IgE im Serum von immunisierten und Kontrolltieren, wurden serielle Verdünnungen der entsprechenden Proben in Mikrotiterplatten, weiche zuvor mit Antimaus-IgE (100 µl/Mulde) als Einfangantikörper überzogen wurden, inkubiert. Nach Blockierung mit PBS mit 1 % BSA und 0,05 % Tween-20 zwei Stunden lang bei Zimmertemperatur wurde eine 1:100 -Verdünnung des Serums in PBS-Tween hinzugefügt (100 µl/Mulde) und die Platten wurden eine Stunde lang bei 37°C inkubiert. Nach dem Spülen wurde biotinyliertes Antimaus-IgE hinzugefügt und die Platten wurden eine weitere Stunde lang bei 37°C inkubiert. Nach viermaligem Spülen wurden 100 µl peroxidase-konjugierten Streptavidins (Pharmingen) in die Mulden gegeben und die Platten wurden 30 Minuten lang bei 37°C inkubiert. Nach vierfachem Spülen wurden die Reaktionen mittels ABTS in 0,1 M Zitratphosphatpuffer (pH 4,35) und einem Gehalt von 0,01 % H₂O₂ entwickelt Es wurden serielle Verdünnungen von gereinigtem Maus-lgE verwendet, um eine Standardkurve zu erzeugen.

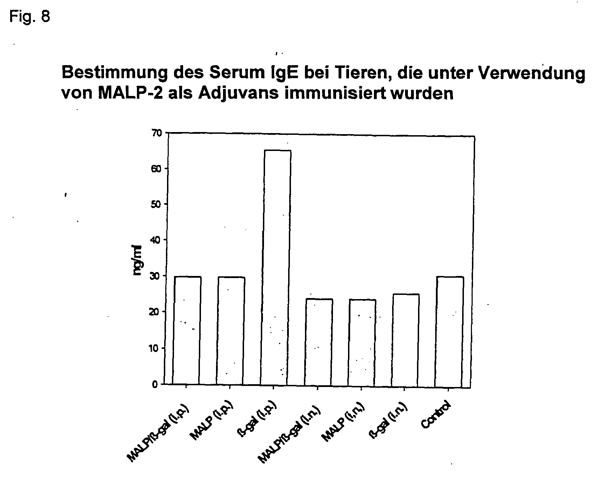

Es ist eine anerkannte Tatsache, dass die Verwendung bestimmter Schleimhautadjuvantien aufgrund einer Erhöhung der Produktion von IgE zu allergischen Reaktionen führen kann. Um diese Hypothese zu überprüfen, haben wir damit begonnen, die Wirkung der Verabreichung von MALP-2 auf den Serumgehalt von IgE zu prüfen. Wie aus Fig. 8 hervorgeht, führt die Administration von MALP-2 über den parenteralen (i.p.) Weg bzw, die Schleimhäute nicht zu einer Erhöhung des Serumgehaltes an IgE. Im Gegenteil, die Anwesenheit von MALP-2 scheint sogar eine positive Wirkung auf die Steigerung des lgE-Gehaltes zu haben wie sie am 28. Tag nach der Impfung mit reiner β-Galactosidase i.p. beobachtet wurde.

### Beispiel 7: MALP-2 stimuliert effektive durch T-Zellen vermittelte Proliferationsreaktlonen, wenn es gemeinsam mit einem löslichen Antigen verabreicht wird

*Versuchsprotokoll:* Unter dem Unterkiefer gelegene Lymphknoten und die Milzen wurden entfernt und für die Analyse der Zellimmunreaktionen zusammengenommen. Die Zellen wurden in RPMI 1640, ergänzt durch 10 % fötales Kalbsserum, 10 U/ml Penizillin, 50 µg/ml Streptomycin, 5 x 10⁻⁵ M 2-Mercaptoethanol und 1 mM L-Glutamin (GIBCO BRL, Karisruhe, Deutschland) vermehrt und bei 37°C in einer feuchten Atmosphäre mit 5 % CO₂ aufbewahrt. Die Suspensionen aus Lymphknoten- und Milzzellen wurden auf 5 × 10⁶ Zellen/ml im vollständigen Medium eingestellt, in eine flachbödige Mikrotiterplatte mit 96 Mulden (Nunc) mit 100 µl/Mulde ausgebracht, und die Platten wurden vier Tage lang in Anwesenheit unterschiedlicher Konzentrationen löslicher ß-Galactosidase inkubiert. Jede Konzentration wurde in Dreiergruppen geprüft. Während der letzten 18 Stunden der Inkubierung wurde jeder Mulde 1 µCi [³H]Thymidin (Amersham International, Freiburg, Deutschland) zugefügt. Anschließend wurden die Zellen auf Filterpapier (Filtermat A; Wallac, Freiburg, Deutschland) unter Verwendung eines Zellemters (Inotech, Wohlen, Schweiz) geerntet und die Menge des eingebetteten [³H]Thymidins in die DNS der vermehrten Zellen wurde mit Hilfe eines γ-Scintillationszählers (Wallac 1450, Micro-Trilux) bestimmt. Die Ergebnisse wurden als das arithmetische Mittel der Aufnahme von [³H]Thymidin in cpm dargestellt.

Die Immunreaktionen von T-Zellen wurden am 31. Tag untersucht, indem die Vermehrung der Zellen, welche aus regionalen Lymphkonten und Milzen nach der in vitro Re-Stimulierung mit β-Galactosidase gewonnen wurden, bestimmt wurde. Die Milzzellen von Tieren, welche mit reiner β-Galactosidase i.p. geimpft worden waren, wurden als positive Kontrollgruppe verwendet und zeigten eine signifikante Vermehrungsreaktion im Vergleich zur nichtimmunisierten Gruppe (Fig. 9A). Eine weitere Steigerung der Proliferation wurde bei Milzzellen festgestellt, welche von Tieren stammten, die eine Co-Injektion von MALP-2 und Antigen erhalten hatten (p < 0,05). Während die i.n. Administration von reiner β-Galactosidase keine nachweisbare Zellvermehrung hervorrief, führte die Mitverabreichung von MALP-2 zu einer effektiven Proliferatiansantwort sowohl bei regionalen (Lymphknotenzellen) als auch bei systemischen (Milzzellen) (Fig. 9B und C). Bemerkenswert ist, dass die stärkste T-Zell-Proliferation bei den Milzzellen von Mäusen beobachtet wurde, bei denen MALP-2 und β-Galactosidase i.n. appliziert worden waren (Fig. 9B). In allen Fällen wurde eine deutlich dosisabhängige Wirkung durch die Erhöhung der β-Galactosidase-Konzentration während der Re-Stimulierung festgestellt (5, 10, 20 µg/ml). Letztlich führte die Verwendung von MALP-2 (0.5 µg) als Adjuvans zu einer statistisch signifikanten (p < 0,05) Steigerung der T-Zellenvermehrung im Vergleich zur i.n. Immunisierung mit (10 µg) plus β-Galactosidase (Fig. 8B).

### Beispiel 8: Analyse von T-Helfermustern hervorgerufen durch die Verwendung von MALP-2 als Adjuvans

*Versuchsprotokoll:* Nunc-Immuno MaxiSorp Testplatten mit 96 Mulden (Nunc, Roskilde, Dänemark) wurden beschichtet mit 100 µl β-Galactosidase (Boehringer, Mannheim, Deutschland) bei 5 µg/ml in 0.05 M Karbonatpuffer (pH 8.2) pro Mulde. Es wurden serielle Zweifachverdünnungen von Serum oder Spülflüssigkeit in PBS mit 1 % BSA und 0,05 % Tween 20 hinzugefügt (100 µl/Mulde), und die Platten wurden 2 Stunden lang bei 37 °C inkubiert. Nach dem Spülen wurde biotinkonjugiertes Ratten-Antimaus-IgG1, -IgG2a, -IgG2b, beziehungsweise -IgG3 (Pharmingen, Hamburg, Deutschland) zugefügt, um die Ig-Unterklassen zu bestimmen. Die Platten wurden eine weitere Stunde lang bei 37°C inkubiert. Nach vierfachem Spülen wurden den Zellen 100 µl peroxidase-konjugierten Streptavidins (Pharmingen) zugefügt, und die Platten wurden 30 Minuten lang bei 37 °C inkubiert. Nach vierfachem Spülen wurden die Reaktionen mit ABTS in 0.1 M Zitratphosphatpuffer (pH 4.35), welcher 0,01 % H₂O₂ enthielt, entwickelt. Um die Konzentration der IgG-Unterklassen im Serum zu bestimmen, wurden Standardkurven erzeugt, indem die Mulden mit einem isotyp-spezifischen Ziegen-Antimaus-IgG beschichtet und dann mit gereinigten Maus-IgG1, -lgG2a, -lgG2b beziehungsweise -lgG3-Antikörpern (Dianova, Hamburg, Deutschland) inkubiert wurden.

Flüssigkeitsüberstände von Kulturen sich vermehrender Zellen wurden an den Tagen 2 und 4 entnommen und bei -70 °C aufbewahrt. Die Bestimmung von IFN-γ IL-2, IL-4, und IL-10 wurde mittels ELISA ausgeführt, wobei kommerzielle Antikörper von Pharmingen gemäß den Herstelleranweisungen verwendet wurden. Kurz gesagt, wurden Mikrotiterplatten mit 96 Mulden über Nacht mit gereinigtem Ratten-Antimaus-IFN-γ, anti-IL-2, anti-IL-4 beziehungsweise anti-IL-10 mAbs (Pharmingen) bei 4 °C beschichtet. Nach drei Spülungen wurden die Platten blockiert und die Flüssigkeitsüberstände wurden in die Mulden gegeben. Für jedes Zytokin wurde eine Standardkurve erzeugt, indem die entsprechenden rekombinanten Nagetierzytokine (Pharmingen) verwendet wurden. Die Platten wurden bei Zimmertemperatur weitere 4 Stunden lang inkubiert. Nach dem Spülen wurde biotinyliertes Ratten-Antimaus-IFN-γ IL-2, IL-4 beziehungsweise -IL-10 mAbs (Pharmingen) in die Mulden gegeben und die Platten wurden eine Stunde lang bei Zimmertemperatur inkubiert. Nach sechsfachem Spülen wurde Streptavidin-Peroxidase-Konjugat zugefügt und die Platten wurden 30 Minuten lang bei Zimmertemperatur inkubiert. Danach wurden die Platten wie oben beschrieben mit ABTS entwickelt.

Zuerst wurde die Unterklassenverteilung des ß-Galactosidase-spezifischen IgG, welches im Serum der immunisierten Mäuse vorhanden war. Wie in Tabelle 1 dargestellt, war der Haupttyp der ß-Galactosidase-spezifischen lgl-Isotypen IgG1, unabhängig vom Immunisierungsprotokoll. Dieses dominante Th2-Reaktionsmuster war schon nach der ersten Impfdosis erkennbar und bleib während der folgenden Booster erhalten. lgG1 war der einzige Isotyp, welcher bei der Verabreichung reiner ß-Galactosidase festgestellt wurde, während die Co-Administration mit CTB oder MALP-2 zur Feststellung weiterer ß-Galactosidase-spezfischer Isotypen, nämlich lgG2a (Typ Th1), lgG2b (Typ Th2) und lgG3 (Typ Th1) führte. Trotzdem blieb das Verhältnis von IgG1/2a (Fig.10), IgG1/2b beziehungsweise IgG1/3 größer als 100.

**Tabelle 1. β-Galactosidase-spezifische IgG-Isotypen im Serum immunisierter Mäuse^{a}**

| Immunisierungsgr uppe | IgG1 | IgG2a | IgG2b | IgG3 |
|---|---|---|---|---|
| β-gal (i.n.) | 22,6 +/- 21,3 | 0,7 +/- 0,5 | 0,3 +/- 0,1 | 0,6 +/- 0,0 |
| β-gal + MALP-2 (i.n.) | 6439,0 +/- 1775, | 20,8 +/- 5,4 | 43,3 +/- 18,9 | 2,4 +/- 0,5 |
| β-gal + CTB (i.n.) | 4108,3 +/- 1437, | 31,9 +/- 9,5 | 49,2 +/- 17,3 | 2,4 +/- 0,6 |
| β-gal (i.p.) | 191,5 +/- 132,1 | 0,1 +/- 0,0 | 0,5 +/- 0,1 | 0,6 +/- 0,0 |
| β-gal + MALP-2 (i.p.) | 2829,7 +/- 1119, | 10,0 +/- 2,8 | 15,8 +/- 6,2 | 2,3 +/- 0,6 |
| Kontrollgruppe | 0,4 +/- 0,0 | 0,23 +/- 0,0 | 0,1 +/- 0,0 | 0,4 +/- 0,0 |

| | | | | |
|---|---|---|---|---|
| ^{a} Die Ergebnisse sind als das Mittel (µg/ml) ± SEM dargestellt (5 Mäuse pro Gruppe) | | | | |

Um die Art der Th-Reaktion, welche durch die Immunisierung hervorgerufen wurde, weiter zu charakterisieren, wurde der Gehalt an IFN-γ, IL-2, IL-4 und lL-10 in den Flüssigkeitsüberständen von *in vitro* stimulierten Milzzellen gemessen (Fig. 11*A*). Es wurde festgestellt, dass von diesen vier Zytokinen IL-10 am stärksten vertreten war, was darauf hinweist, dass ein Th2-Antwortmuster erzeugt worden war. Der Gehalt an IL-10 war bei Mäusen, welche mit MALP-2 i.n. immunisiert worden waren, signifikant höher, als bei der Kontrollgruppe (2,2 ng/ml im Vergleich zu 0,009 ng/ml, p < 0,005) und auch im Vergleich zu Tieren, bei denen CTB als Schleimhautadjuvans verwendet worden war (0,6 ng/ml, p < 0,05). Das beobachtete Antwortmuster mit einem dominanten Zytokin vom Typ Th2 stimmte mit der Feststellung von β-Galactosidase-spezifischem IgG1 bei denselben Tieren überein (Fig. 10). Tatsächlich stimmt die starke Stimulierung der IL-10-Sekretion mit der Rolle überrein, welche dieses Zytokin bei der Hemmung der Zytokinsynthese durch Th1-Zellen, der Verbesserung der B-Zellenvermehrung und der Stimulierung der IgA-Produktion spielt.

Trotz der Tatsache, dass in den Zelikulturmedien von Zellen, die aus regionalen Lymphknoten gewonnen wurden, geringere absolute Werte der Zytokinkonzentration festgestellt wurden, glich das allgemeine Muster dem für die Milzzellkulturen festgestellten (Fig. 10). Interessant ist, dass zwar die Ausscheidung von 1L-10 und IL-4 auch in den Zellen von Mäusen stimuliert wurde, die mit reiner β-Galactosidase i.p. immunisiert worden waren, die Th1-Zytokine IL-2 und IFN-γ aber unter der Nachweisgrenze blieben. Im Gegensatz dazu wurden IL-2 und IFN-γ auch bei den Mäusen festgestellt, welche das Antigen gemischt mit CTB oder MALP-2 erhalten hatten (Fig. 10). Diese Ergebnisse stimmen überein mit den festgestellten IgG-Isotypmustem (Tabelle 1) und bestätigen, dass zwar Th2-Reaktionstypen vorherrschen, aber MALP-2 auch die Stimulierung von Th1-Zellen unterstützt.

### Beispiel 9: Orale Immunisierung mit dem Modellantigen ß-Galactosidase unter Verwendung von MALP-2 als Schleimhautadjuvans.

Es wurden sechs bis acht Wochen alte weibliche BALB/c (H-2d)- Mäuse von Harlan Winkelmann GmbH (Borchen, Deutschland) eingekauft und entsprechend lokaler Richtlinien und Richtlinien der europäischen Gemeinschaft behandelt Gruppen von je 5 Mäusen wurden an Tagen 1, 14, 21 und 31 mit 100 µg β-gal (Boehringer, Mannheim, Deutschland) entweder alleine oder mit 2 µg synthetischem MALP-2 als Adjuvans auf oralem Wege (Dosis 25 µl) immunisiert. An Tag 45 wurden Serumproben genommen und bei - 20 °C bis zur Bestimmung β-gal-spezifischer Antikörper gelagert. 96-muldige Nunc-lmmuno MaxiSorp® Testplatten (Nunc, Roskilde, Dänemark) wurden beschichtet mit 100 µl β-gal zu 5 µg/ml in 0,05 M Carbonatpuffer (pH 8,2) pro Mulde. Es wurden serielle zweifache Verdünnungen der Seren oder Lavagen in PBS mit 1 % BSA und 0,05 % Tween 20 zugegeben (100 µl/Mulde), und die Platten wurden 2 Stunden bei 37 °C inkubiert. Nach dem Waschen wurde blotinyliertes γ-Ketten-spezifisches Ziege-anti-Maus IgG (Sigma Chemie, Deisenhofen, Deutschland) zugegeben, und die Platten wurden eine weitere Stunde bei 37 °C inkubiert. Nach viermaligem Waschen wurde den Zellen 100 µl peroxidasekonjugiertes Streptavidin (Pharmingen) zugegeben, und die Platten wurden 30 Minuten bei 37 °C inkubiert. Nach vier Waschvorgängen wurden Reaktionen mit ABTS in 0,1 M Citrat-Phosphat-Puffer (pH 4,35), der 0,01 % H₂O₂ enthielt, entwickelt. Die Endwert-Titer wurden als die rezlproken Iogharithmischen log₂-Werte der letzten Verdünnung ausgedrückt, was nach 30 Minuten Inkubieren eine optische Dichte bei 405 nm von 0,1 Einheiten über den Werten der Negativkantrollen ergab. Immunantworten gegen β-gal wurden nur bei Tieren beobachtet, die mit MALP-2 als Schleimhautadjuvans immunisiert bzw. geimpft worden waren (siehe Fig. 12).

Es ist zu erwarten, dass auch bei unterschiedlichen Dosierungen Immunreaktionen sowohl in den Körperflüssigkeiten als auch in den Zellen hervorgerufen werden, welche wirksamer sind, als die von der alleinigen Verabreichung des Antigens hervorgerufenen. Darüber hinaus sind antigenspezifische Schieimhautreaktionen zumindest lokal In den Därmen (d.h. das Vorhandensein von antigenspezifischem sekretorischen IgA in Darmausspülungen) festzustellen. Außerdem ist zu erwarten, dass sekretorische Reaktionen in entfernten Schleimhäuten festgestellt werden können.

## Patentansprüche

1. Verwendung eines Lipopeptids oder Lipoproteins der Struktur (I) 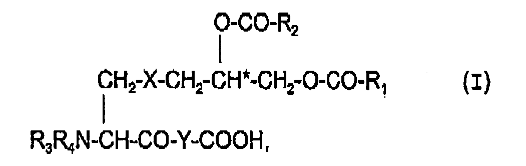 wobei
R₁ und R₂, die gleich oder voneinander verschieden sein können, für C₇₋₂₅-Alkyl, C₇₋₂₅-Alkenyl oder C₇₋₂₅-Alkinyl,
X für S, O oder CH₂,
R₃ und R₄ unabhängig voneinander für H oder Methyl und
Y für eine aus 1 bis 25, vorzugsweise 12 bis 25 Aminosäureresten bestehende physiologisch verträgliche und in der verwendeten Spezies nicht per se immunogene Aminosäuresequenz stehen,
und das mit * markierte asymmetrische Kohlenstoffatom gemäß der Regel von Cahn-Ingold-Prelog die absolute R-Konfiguration hat, wenn X = S (Schwefel) ist,
als Mufcosal-Adjuvans bei therapeutischer oder prophylaktischer Vakzinierung über die Schleimhäute.

2. Verwendung nach Anspruch 1, **dadurch gekennzeichnet, dass** die Aminosäuresequenz Y vorzugsweise ausgewählt ist aus.
a) GQTNT
b) SKKKK
c) GNNDESNISFKEK
d) GQTDNNSSQSAAPGSGTTNT.

3. Verwendung nach Anspruch 1 oder 2, **dadurch gekennzeichnet, dass** das Lipoprotein oder Lipopeptid gemäß Struktur (I) ein S-[2,3-Bispalmitoyloxy-(2*R*)-propyl] cysteinyl-peptid ist, wobei das Peptid eine aus 1 bis 25 Aminosäureresten bestehende physiologisch verträgliche, in der verwendeten Spezies vorzugsweise nicht immunogene Aminosäuresequenz ist.

4. Verwendung nach einem der Ansprüche 1 bis 3, **dadurch gekennzeichnet, dass** das Mucosal-Adjuvans in einer Zubereitung mit der eigentlichen Vakzin-Komponente vorliegt, die für intranasale, intra-NALT, aerosoliert orale, intrarektale, conjunctivale, intravaginale oder intraurethrale Applikation oder eine Applikation in die Milchgänge der weiblichen Brust vorgesehen ist

5. Verwendung nach einem der Ansprüche 1 bis 3, **dadurch gekennzeichnet, dass** das Mucosal-Adjuvans in einem Kit für die Co-Applikation mit einem Vakzin in die Milchgänge der weiblichen Brust, auf intranasalem, intra-NALT, aersolisiert oralem, intrarektalem, conjunctivalem, intravaginalem oder intraurethralem Wege vorliegt.

6. Verwendung eines Lipopeptids oder Lipoproteins der allgemeinen Struktur (I) 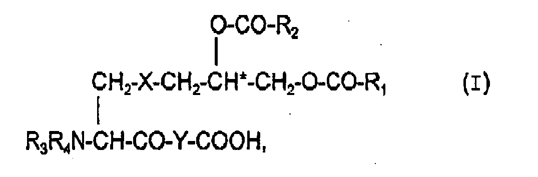 wobei
R₁ und R₂, die gleich oder voneinander verschieden sein können, für C₇₋₂₅-Alkyl, C₇₋₂₅-Alkenyl oder C₇₋₂₅-Alkinyl,
X für S, O oder CH₂,
R₃ und R₄ unabhängig voneinander für H oder Methyl und
Y für eine aus 1 bis 25 Aminosäureresten, vorzugsweise 12 bis 25 Aminosäureresten, bestehende physiologisch verträgliche und in der verwendeten Spezies nicht per se immunogene Aminosäuresequenz stehen, und das mit * markierte asymmetrische Kohlenstoffatom gemäß der Regel von Cahn-Ingold-Prelog die absolute R-Konfiguration hat, wenn X = S (Schwefel) ist,
ausgenommen eines S-(2,3-Diacyloxypropyl)cystein-peptids der Sequenz DhcGNNDESNISFKEK, wobei N-terminal die Aminosäuren an Positionen 2 und gegebenenfalls 3 fehlen und/oder C-terminal 1 bis 2 Aminosäuren deletiert sein können,
als Adjuvans bei einer nicht mukosalen Vakzinierung.

7. Verwendung nach einem der Ansprüche 1 bis 6, **dadurch gekennzeichnet, dass** das Lipopeptid oder Lipoprotein in einer Zubereitung mit wenigstens einem weiteren Adjuvans und/oder Antigen vorliegt.

8. Verwendung nach einem der Ansprüche 1 bis 7, **dadurch gekennzeichnet, dass** das Lipopeptid oder Lipoprotein mit einem physikalischen oder biologischen Träger assoziiert oder verbunden ist.

9. Verwendung nach einem der Ansprüche 1 bis 8, **dadurch gekennzeichnet, dass** das Lipopeptid oder Lipoprotein mit ein oder mehreren entzündungshemmenden, anti-angiogenen, cytotoxischen oder immunmodulatorischen Stoffen oder Liganden oder mit Antikörpern gemeinsam verabreicht wird oder in einer Zubereitung mit diesen vorliegt.

10. Verwendung nach einem der Ansprüche 1 bis 9, **dadurch gekennzeichnet, dass** das Lipopeptid oder Lipoprotein in einer Zubereitung vorliegt, die weitere Zusatz- und Hilfsstoffe, insbesondere Konservierungsstoffe oder Stabilisatoren enthält.

11. Verwendung nach einem der Ansprüche 1 bis 10, **dadurch gekennzeichnet, dass** das Vakzin, das durch das Adjuvans begleitet wird, in Form von Peptiden, Proteinen, DNA, Polysacchariden, Glycolipiden oder Glykoproteinen.
